# EUROPEAN PATENT SPECIFICATION

(11) **EP 2 910 012 B1**
(45) Date of publication and mention of the grant of the patent: **12.06.2024**
(21) Application number: 13847334.3
(22) Date of filing: 18.10.2013
(51) Int. Cl.: G01J 3/02, G01J 3/28, G01J 3/36, A61B 5/00, A61B 5/1455, A61B 5/145, H04N 23/11, H04N 23/56

(54) **SINGLE-SENSOR HYPERSPECTRAL IMAGING DEVICE**
HYPERSPEKTRALE EINZELSENSOR-BILDGEBUNGSVORRICHTUNG
DISPOSITIF D'IMAGERIE HYPERSPECTRALE À CAPTEUR UNIQUE

(30) Priority: 19.10.2012 US 201261716401 P; 15.03.2013 US 201313844737
(43) Date of publication of application: 26.08.2015
(73) Proprietor: Samsung Electronics Co., Ltd., Gyeonggi-do 16677 (KR)
(72) Inventor: DARTY, Mark, Anthony, Greenwich, CT 06830 (US)
(74) Representative: Grünecker Patent- und Rechtsanwälte PartG mbB
(86) International application number: PCT/US2013/065785
(87) International publication number: WO 2014/063117

(56) References cited:
- WO-A2-2007/008826
- WO-A2-2007/022508
- US-A- 5 568 186
- US-A1- 2006 274 308
- US-A1- 2007 016 079
- US-A1- 2009 268 045
- US-A1- 2010 140 461
- US-A1- 2011 141 569
- US-B2- 6 831 688
- US-B2- 7 589 772
- Anonymous: "Pulse oximetry - Wikipedia", , 5 October 2012 (2012-10-05), XP093005621, Retrieved from the Internet: URL:https://en.wikipedia.org/w/index.php?t itle=Pulse_oximetry&oldid=516141642 [retrieved on 2022-12-07]
- LOSSON O ET AL: ""Chapter 5 - Comparison of Color Demosaicing Methods"", ADVANCES IN IMAGING AND ELECTRON PHYSICS, ELSEVIER SCIENCE & TECHNOLOGY, SAN DIEGO, CALIF. [U.A.] : ACAD. PRESS, US, vol. 162, 1 January 2010 (2010-01-01), pages 173-265, XP009192124, ISSN: 1076-5670, DOI: 10.1016/S1076-5670(10)62005-8 ISBN: 978-0-12-014738-0
- Svetlana V. Panasyuk ET AL: "Medical hyperspectral imaging to facilitate residual tumor identification during surgery", Cancer biology & therapy, vol. 6, no. 3, 27 March 2007 (2007-03-27), pages 439-446, XP055315127, US ISSN: 1538-4047, DOI: 10.4161/cbt.6.3.4018

## Description

### RELATED APPLICATIONS

This application claims the benefit of U.S. Provisional Patent Application No. 61/716,401 filed on October 19, 2012, and U.S. Patent Application No. 13/844,737, filed March 15, 2013.

### TECHNICAL FIELD

The present disclosure relates to hyperspectral spectroscopy, and in particular, to systems, methods and devices enabling a single-sensor hyperspectral imaging device.

### BACKGROUND

Hyperspectral (also known as "multispectral") spectroscopy is an imaging technique that integrates multiple images of an object resolved at different spectral bands (e.g., ranges of wavelengths) into a single data structure, referred to as a three-dimensional hyperspectral data cube. Hyperspectral spectroscopy is often used to identify an individual component of a complex composition through the recognition of corresponding spectral signatures of the individual components in a particular hyperspectral data cube.

Hyperspectral spectroscopy has been used in a variety of applications, ranging from geological and agricultural surveying to military surveillance and industrial evaluation. Hyperspectral spectroscopy has also been used in medical applications to facilitate complex diagnosis and predict treatment outcomes. For example, medical hyperspectral imaging has been used to accurately predict viability and survival of tissue deprived of adequate perfusion, and to differentiate diseased (e.g. tumor) and ischemic tissue from normal tissue.

However, despite the potential clinical value of hyperspectral imaging, several drawbacks have limited the use of hyperspectral imaging for medical diagnostics. In particular, current medical hyperspectral instruments are costly because of the complex optics and computational requirements currently used to capture and process images at a plurality of spectral bands in order to generate a satisfactory hyperspectral data cube. Previously available hyperspectral imaging instruments also often suffer from poor temporal and spatial resolution, as well as low optical throughput, due to the complex optics and taxing computational requirements needed for assembling, processing, and analyzing data in order to generate a hyperspectral data cube satisfactory for medical use.

US 2010/140461 A1 relates to multispectral filter arrays and methods of making and using the arrays are described herein. Multispectral imaging systems and methods of making and using the systems are also described. A multispectral filter array includes a mosaic of light sensitive elements that includes a first element sensitive to a first narrow spectral region, a second element sensitive to a second narrow spectral region, and a third element sensitive to a third narrow spectral region. A multispectral imaging system includes an illumination system having at least three lighting elements, each of which are configured to transmit light at a different wavelength, and the multispectral filter array. Further prior art hyperspectral imaging devices and their use in medical diagnosis are disclosed in WO2007/022508A2, Svetlana V. Panasyuk ET AL: "Medical hyperspectral imaging to facilitate residual tumor identification during surgery", Cancer biology & therapy, vol. 6, no. 3, pages 439-446, and US2007/016079A1.

### SUMMARY

It is the object of the present invention to improve the determination of the concentration of one or more skin or blood components, in order to determine the probability that a region of a subject is afflicted with one of a first and second medical conditions.

This object is solved by the subject matter of the independent claims. Preferred embodiments of the present invention are defined by the dependent claims.

Various implementations of systems, methods and devices within the scope of the appended claims each have several aspects, no single one of which is solely responsible for the desirable attributes described herein. Without limiting the scope of the appended claims, some prominent features are described herein. After considering this discussion, and particularly after reading the section entitled "Detailed Description" one will understand how the features of various implementations are used to enable a hyperspectral imaging device capable of producing a three-dimensional hyperspectral data cube using a single photo-sensor chip (e.g. CDD, CMOS, etc) suitable for use in a number for applications, and in particular, for medical use.

One aspect of the present disclosure provides a hyperspectral imaging device comprising a photo-sensor array including a plurality of photo-sensors. Each photo-sensor provides a respective output. The device further comprises a spectral filter array having a plurality of filter elements. Each filter element is arranged to filter light received by a respective one or more of the photo-sensors. Each filter element is one of a plurality of filter-types. Each filter-type characterized by a unique spectral pass-band. The device further comprises an interface module to select a plurality of subsets of photo-sensor outputs. Each such subset is associated with a single respective filter-type. The device comprises a control module that generates a hyperspectral data cube from the subsets of photo-sensor outputs by generating a plurality of images. Each such image is produced from a single corresponding subset of photo-sensor outputs in the plurality of photo-sensor outputs and so is associated with a corresponding filter-type in the plurality of filter-types.

According to claim 1, the controller is further configured to capture single frame image data by controlling the exposure of the combination of the photo-sensor array and spectral filter array to light. In all embodiments the hyperspectral data cube is generated from the single frame image data, and each respective image in the plurality of images is generated by applying an interpolation process to the corresponding subset of photo-sensor outputs for the filter-type corresponding to the respective image.

The plurality of filter elements comprises a first filter element and a second filter element of the same filter-type and a center-to-center distance between the first filter element and the second filter element is less than 250 microns, preferably less than 200 microns, less than 150 microns, less than 100 microns or less than 50 microns.

The filter elements of a first filter-type in the plurality of filter-types are spatially distributed across the spectral filter array. For instance, in some embodiments, this spatial distribution of the filter elements of the first filter-type is a uniform distribution throughout the spectral filter array.

In some embodiments, a spatial distribution of the filter elements in the plurality of filter elements is characterized by a repeating pattern of one or more filter-types.

In some embodiments, the plurality of filter-types includes at least three filter-types, at least four filter-types, at least five filter-types, at least six filter-types, at least seven filter-types, at least eight filter-types, at least nine filter-types, at least ten filter-types, at least fifteen filter-types, at least twenty filter-types, at least twenty-five filter-types, or at least thirty filter-types.

In some embodiment the interface module comprises circuitry configured to select the one or more subsets of photo-sensor outputs. In some embodiments, the interface module comprises a plurality of registers configured to receive the output of the photo-sensor array and the control module is further configured to identify which registers in the plurality of registers correspond to filter elements of a particular filter-type in the plurality of filter-types using a look-up table. The control module selects one or more subsets of photo-sensor outputs from the plurality of registers based on the identification of the registers that correspond to filter elements of the particular filter-type. In some such embodiments, the control module is also operable to bundle photo-sensor outputs for the particular filter-type into data packets, where the data packets include at least the register values of the registers that include data for the particular filter-type. In some such embodiments, the hyperspectral imaging device further comprises a transceiver to transmit the data packets to a server, and receive an image for each filter-type from the server based on the transmitted data packets.

In some embodiments, the hyperspectral imaging device is handheld.

Another aspect of the present disclosure, which does not form part of the claimed invention, provides a method for forming a hyperspectral imaging cube in which there is selected, from a photo-sensor array comprising a plurality of photo-sensors, a first subset of photo-sensor outputs from a first subset of photo-sensors in the plurality of photo-sensors. Each photo-sensor in the first subset of photo-sensors is filtered by a filter, in a plurality of filters, of a first filter-type in a plurality of filter-types. Each such filter-type in the plurality of filter-types is characterized by a spectral pass-band different from the other filter-type. A first image is formed using the first subset of photo-sensor outputs.

A second subset of photo-sensor outputs from a second subset of photo-sensors in the plurality of photo-sensors is also selected. Each photo-sensor in the second subset of photo-sensors is filtered by a filter of a second filter-type in the plurality of filter-types. A second image is formed using the second subset of photo-sensor outputs.

A third subset of photo-sensor outputs from a third subset of photo-sensors in the plurality of photo-sensors is also selected. Each photo-sensor in the third subset of photo-sensors is filtered by a filter of a third filter-type in the plurality of filter-types. A third image is formed using the third subset of photo-sensor outputs; and

The hyperspectral imaging cube is formed using the first image, the second image and the third image. The first image, the second image and the third image each represent the same area of an object and the first image, the second image and the third image are each characterized by different wavelengths or wavelength ranges.

In all embodiments the photo-sensor array is subjected to a single common exposure of light in order to concurrently generate the first subset of photo-sensor outputs, the second subset of photo-sensor outputs, and the third subset of photo-sensor outputs.

In all embodiments, the first subset of photo-sensors comprises a photo-sensor covered by a filter first element and second photo-sensor covered by a second filter element, where a center-to-center distance between the first filter element and the second filter element is less than 250 microns, preferably less than 200 microns, less than 150 microns, less than 100 microns or less than 50 microns and where the first filter element and the second filter element are both the same filter-type.

In some embodiments, the first subset of photo-sensors is spatially distributed across the spectral filter array, e.g., as a uniform distribution across the photo-sensor array.

In some embodiments, a spatial distribution of filters in the plurality of filters of the same filter type is a uniform distribution across the photo-sensor array.

In some embodiments, a spatial distribution of filters in the plurality of filters is characterized by a repeating patterned based on filter type.

In some embodiments, the hyperspectral imaging cube produced by the method comprises four or more images, five or more images, six or more images, seven or more images, eight or more images, nine or more images, ten or more images, fifteen or more images, twenty or images, twenty-five or more images, or thirty or more images, each respective image representing the same area of the object at a different wavelength or wavelength range.

### BRIEF DESCRIPTION OF THE DRAWINGS

So that the present disclosure can be understood in greater detail, a more particular description may be had by reference to aspects of various implementations, some of which are illustrated in the appended drawings. The appended drawings, however, merely illustrate the more pertinent aspects of the present disclosure and are therefore not to be considered limiting, as the description may admit to other effective aspects and arrangements.
Figure 1 is an example of a distributed diagnostic environment including single-sensor hyperspectral imaging devices in accordance with an embodiment of the present disclosure.
Figure 2 is a schematic diagram of a local diagnostic environment in accordance with an embodiment of the present disclosure.
Figure 3 is a detailed diagram of an example implementation of a single-sensor hyperspectral imaging device in accordance with an embodiment of the present disclosure.
Figure 4 is an exploded schematic view of an implementation of an image sensor assembly in accordance with an embodiment of the present disclosure.
Figure 5 is a block diagram of an implementation of a single-sensor hyperspectral imaging device in accordance with an embodiment of the present disclosure.
Figure 6 is a schematic illustration of a hyperspectral data cube in accordance with an embodiment of the present disclosure.
Figure 7 is a flowchart representation of an implementation of a method associated with a single-sensor hyperspectral imaging device in accordance with an embodiment of the present disclosure.

In accordance with common practice the various features illustrated in the drawings may not be drawn to scale. The dimensions of various features may be arbitrarily expanded or reduced for clarity. In addition, some of the drawings may not depict all of the components of a given system, method or device. Finally, like reference numerals may be used to denote like features throughout the specification and figures.

### DETAILED DESCRIPTION

Numerous details are described herein in order to provide a thorough understanding of the example implementations illustrated in the accompanying drawings. However, the invention may be practiced without many of the specific details. Well-known methods, components, and circuits have not been described in exhaustive detail so as not to unnecessarily obscure more pertinent aspects of the implementations described herein.

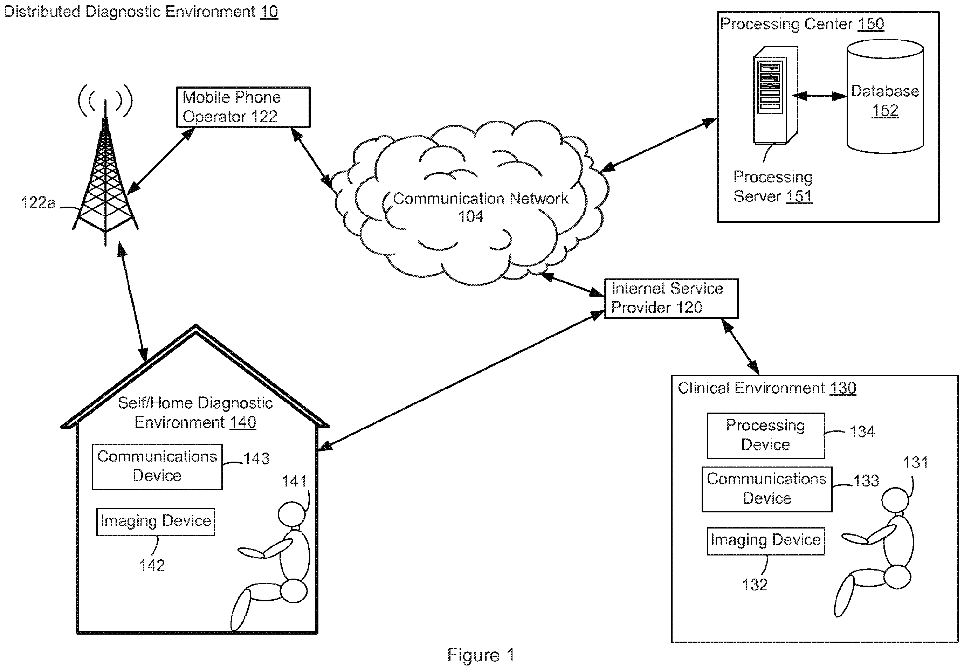

Figure 1 is an example of a distributed diagnostic environment 10 including a single-sensor hyperspectral imaging device 132/142 according to some implementations.

In some implementations, the distributed diagnostic environment 10 includes one or more clinical environments 130, one or more self/home diagnostic environments 140, one or more processing centers 150, and a communication network 104 that, together with an Internet Service Provider 120 and/or Mobile phone operator 122, with concomitant cell towers 122a, allow communication between the one or more environments 130/140 and the one or more processing centers 150.

Turning to the self/home diagnostic environment 140 depicted in Figure 1, an advantage of the present disclosure is that the hyperspectral imager is small and portable, allowing for the realization of the disclosed environment 140. The self/home diagnostic environment 140 includes an imaging device 142 and a communications device 143. The communications device 143 communicates with processing center 150 via communications network 140.

In some implementations, the imaging device 142 illuminates an object (e.g., an area of the body of a subject 141) and generates imaging data of the object. In some implementations, the imaging device 142 illuminates an object using one or more light sources (not shown). In some implementations, after illuminating the object, or concurrently thereto, the imaging device 142 generates and transmits imaging data (e.g., the hyperspectral image data set) corresponding to the object to processing center 150 for forming a processed hyperspectral image. In other implementations, the imaging device 142 forms the processed hyperspectral image using the hyperspectral image data set, and transmits the processed hyperspectral image to the processing center 150.

The clinical environment 130 depicted in Figure 1 is similar to the self/home diagnostic environment 140. The exception is that the clinical environment 130 is designed to test several patients 131. To accommodate this demand, in some embodiments, the clinical environment 130 includes a processing device 134 for processing hyperspectral images without reliance on processing center 150. As such, in some embodiments, the clinical environment 130 includes the processing device 134, a communications device 133, and an imaging device 132. The communications device 133 communicates with processing center 150 via communications network 140.

In some implementations, the imaging device 132 illuminates an object (e.g., an area of the body of a patient 131) and generates imaging data of the object. In some implementations, the imaging device 132 illuminates an object using one or more light sources (not shown). In some implementations, after illuminating the object, or concurrently thereto, the imaging device 132 generates and transmits imaging data (e.g., the hyperspectral image data set) corresponding to the object to processing center 150 for forming a processed hyperspectral image. In other implementations, the imaging device 132 transmits the hyperspectral image data set to processing device 134 where the processed hyperspectral image is formed using the hyperspectral image data. In some embodiments, processing device 134 is a desktop computer, a laptop computer, and/or a tablet computer. In still other implementations, the imaging device 132 forms the processed hyperspectral image using the hyperspectral image data set, and transmits the processed hyperspectral image to the processing center 150 via communications device 133.

In some implementations, prior to transmitting the hyperspectral imaging data set, the imaging device 132 transforms the imaging data by performing at least one of adjusting the brightness of at least one of the respective digital images in the hyperspectral imaging data (e.g., image 1337-1-N at wavelength range No. N), adjusting the contrast of at least one of the respective digital images in the hyperspectral imaging data, removing an artifact from at least one of the respective digital images in the hyperspectral imaging data, cropping at least one of the respective digital images in the hyperspectral imaging data, processing one or more sub-pixels of at least one of the respective digital images in the hyperspectral imaging data, compressing the size of at least one of the respective digital images in the hyperspectral imaging data, assembling a plurality of digital images in the hyperspectral imaging data into a hyperspectral data cube, transforming a hyperspectral data cube, formatting data contained within at least one of the respective digital images in the hyperspectral imaging data, and encrypting data contained within at least one of the respective digital images in the hyperspectral imaging data.

The processing center 150 depicted in Figure 1 receives images from self/home diagnostic environment and/or clinical environment 130 and processes them using processing server 151 before storing them using database 152 for subsequent retrieval.

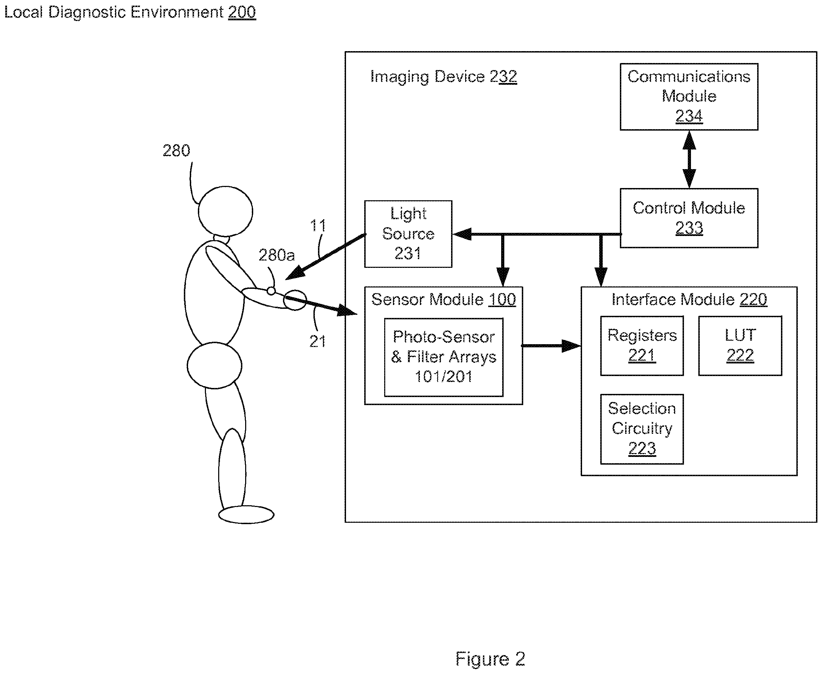

Figure 2 is a schematic diagram of a local diagnostic environment 200 according to some implementations. Local diagnostic environment 200 differs from distributed diagnostic environment in the sense that there is no requirement that the local diagnostic environment make use of a processing center 150 for the storage and/or processing of hyperspectral images. The local diagnostic environment 200 includes an imaging device 232 and a communications module 234. The communications module 234 is used, for example, to optionally communicate hyperspectral imaging data to a remote location and/or to receive software updates or diagnostic information.

In some implementations, the imaging device 232 illuminates an object (e.g., an area 280a of the body of a subject 280) and generates imaging data of the object. In some implementations, the imaging device 232 illuminates an object using one or more light sources (231). Such light sources emit light 11 that is reflected by area 280a to form reflected light 21 that is received by sensor module 100. Sensor module 100 includes photo-sensor and filter arrays 101/201.

In some embodiments, output of the photo-sensor and filter arrays 101/201 is sent to registers 221 of an interface module 220 and processed by one or more register look-up tables 222 and selection circuitry 223. For instance, in some embodiments, look-up table 222 is used in the following manner. In such embodiments, for purposes of illustration, registers 221 is a plurality of registers. The hyperspectral imaging device 232 uses the registers 221 to receive the output of the photo-sensor array 101 and the control module 223 identifies which registers 221 in the plurality of registers correspond to filter elements of a particular filter-type in a plurality of filter-types using the look-up table. The control module 223 selects one or more subsets of photo-sensor outputs from the plurality of registers based on the identification of the registers that correspond to filter elements of the particular filter-type. The independent subsets of photo-sensors are then used to form independent images, each image corresponding to a filter-type.

Operation of the light source 231, sensor module 100 and interface module 220 is under the control of control module 233. In some embodiments, as illustrated in Figure 2, control module 233, in turn, interacts with a communications module 234 in order to facilitate the acquisition of hyperspectral imaging data from a subject 280.

In various embodiments, light sources emitting radiation in the ultraviolet spectrum (wavelengths from about 10 nm to about 400 nm), visible spectrum (wavelengths from about 400 nm to about 760 nm), and/or near-infrared spectrum (wavelengths from about 760 nm to about 2000 nm) are used in the hyperspectral/multispectral imaging systems and methods provided herein.

In some implementations, light source 231 includes one or more broadband light sources, one or more narrowband light source, or a combination of one or more broadband light source and one or more narrowband light source. In some implementations, light source 231 includes one or more coherent light sources, one or more incoherent light sources, or a combination of one or more coherent and one or more incoherent light sources.

In some implementations, light source 231 includes one or more narrow bandwidth LED lights. In one implementation, the one or more narrowband LED lights have a FWHM spectral bandwidth or less than about 100 nm, preferably less than about 50 nm, more preferably less than 25 nm. In one implementation, light source 231 includes one or more LED source that emits radiation in the infrared, preferably near-infrared, spectrum. The used of near-infrared LED illumination in is commonly found in closed circuit security cameras. For additional information on light emitting diodes, *see,* Schubert E.F., Light Emitting Diodes, Second Edition, Cambridge University Press (2006), the content of which is hereby incorporated herein by reference in its entirety for all purposes.

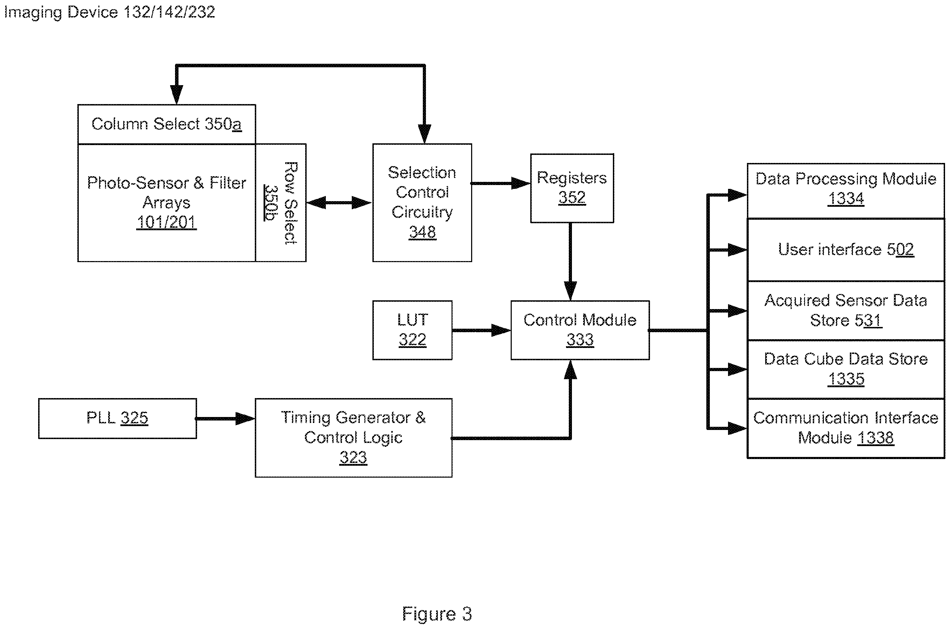

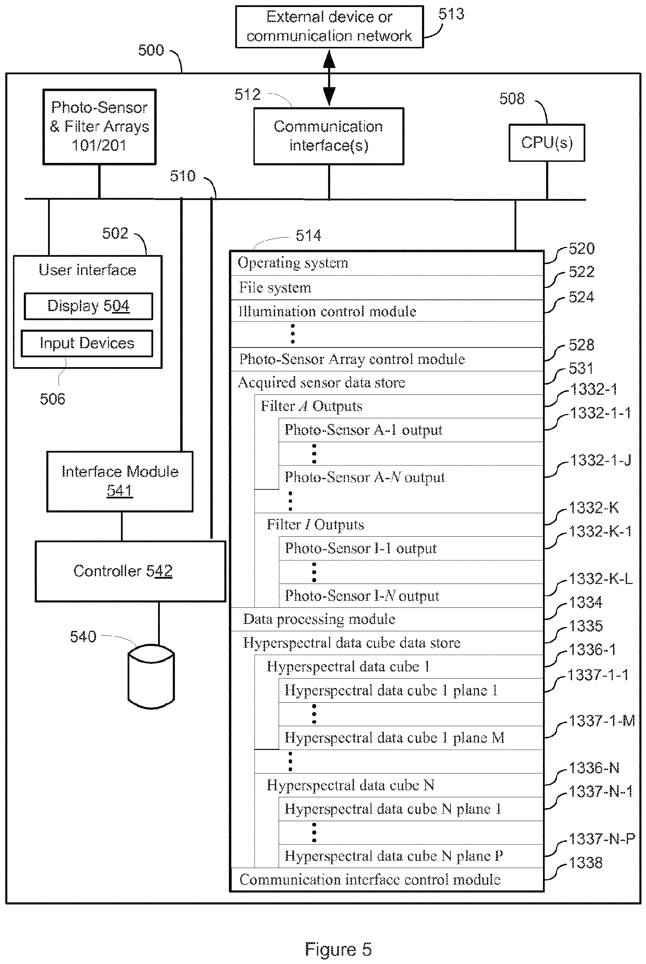

Figure 3 is a detailed diagram of an example implementation of a single-sensor hyperspectral imaging device 132/142/232 in accordance with the present disclosure. In general, the timing generator and control logic 323 controls frame exposure mode timing, frame rate adjustment, and frame rate timing. In some embodiments, timing generator and control logic 323 relies on phased-lock loop 325 (PLL) for timing signals. These aforementioned components work in conjunction with a control module 333 and look-up table 322 to control acquisition of images from the photo-sensor and filter arrays 101/102. To this end, there is selection control circuitry 348 to select data using column select 350a and row select 350b circuitry. This data is stored and processed in registers 352. This data is passed, under the direction of the control module 333 to data processing module 1334 which worked in conjunction with user interface 502, acquired sensor data store 531, data cube data store 1335, and communication interface module 1338. These modules, interfaces and data stores are described in more detail below on conjunction with Figure 5.

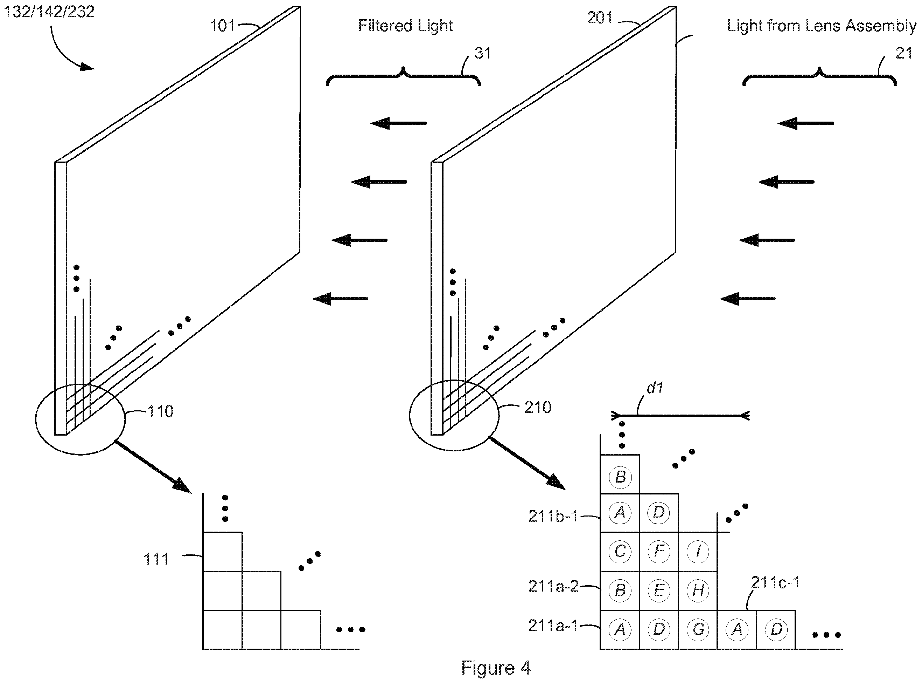

Figure 4 is an exploded schematic view of an implementation of an image sensor assembly for a single-sensor hyperspectral imaging device 132/142/232. The image sensor assembly 100 includes a photo-sensory array 101 in combination with a filter array 201. While some example features are illustrated in Figure 4, those skilled in the art will appreciate from the present disclosure that various other features have not been illustrated for the sake of brevity and so as not to obscure more pertinent aspects of the example implementations disclosed herein. For example, the various electrical connections and access control circuitry to receive the outputs of the photo-sensor array 101 have not been illustrated. Nevertheless, those skilled in the art will appreciate that at least one of various configurations of electrical connections and access control circuitry to receive the outputs of the photo-sensor array 101 would be included in an operable single-sensor hyperspectral imaging device. Moreover, an interface module and a controller - which are together configured to select, assemble, process, and analyze the outputs of the photo-sensor array 101 into a hyperspectral data cube - are described above with reference to Figure 3.

With further reference to Figure 4, the photo-sensory array 101 includes a plurality of photo-sensors. For example, detailed view 110 schematically shows, as a non-limiting example only, a number of photo-sensors 111 included in the photo-sensor array 101. Each photo-sensor 111 generates a respective electrical output by converting light incident on the photo-sensor.

In some implementations, the photo-sensor array 101 includes a CCD (charge coupled device) semiconductor sensor array. A CCD sensor is typically an analog device. When light strikes a CCD sensor array, the light is converted to and stored as an electrical charge by each photo-sensor. The charges are converted to voltage, on a photo-sensor by photo-sensor basis, as they are read from the CCD sensor array. Often, but not exclusively, one photo-senor is synonymous with a respective single pixel. However, in various implementations, a single pixel is configured to include two or more pixels.

In some implementations, the photo-sensor array 101 includes a CMOS (complementary metal oxide) semiconductor sensor array. A CMOS photo-sensor is an active photo-sensor that includes a photodetector and an active amplifier. In other words, each photo-sensor in a CMOS sensor array includes a respective photodetector and a corresponding active amplifier.

In some implementations, the photo-sensor array 101 includes a hybrid CCD/CMOS sensor array. In some implementations, a hybrid CCD/CMOS sensor array includes CMOS readout integrated circuits (ROICs) that are bump bonded to a CCD imaging substrate. In some implementations, a hybrid CCD/CMOS sensor array is produced by utilizing the fine dimensions available in modem CMOS technology to implement a CCD like structure in CMOS technology. This can be achieved by separating individual polysilicon gates by a very small gap.

The light incident on a particular photo-sensor 111 is filtered by a respective filter in the filter array 201. In all embodiments of claim 1, the filter array 201 is configured to include a plurality of filter elements. Each filter element is arranged to filter light received by a respective one or more of the plurality of photo-sensors in the photo-sensor array 101. Each filter element is also one of a plurality of filter-types, and each filter-type is characterized by a spectral pass-band different from the other filter-types. As such, the electrical output of a particular photo-sensor is associated with a particular spectral pass-band associated with the respective filter associated the particular photo-sensor 111.

For example, the detailed view 210 schematically shows, as a non-limiting example only, a number of filter-types *A, B, C, D, E, F, G, H,* and *I* are included in the filter array 201. In one implementation, at least two of filter types *A, B, C, D, E, F, G, H,* and *I* have different spectral pass-bands. For example, as illustrated in Figure 4, filter elements 211a-1 and 211a-2 of filter types *A* and *B,* respectively, have different spectral pass-bands. In some implementations, at least two of filter types *A, B, C, D, E, F, G, H,* and *I* have the same spectral pass-band and at least two of filter types *A, B, C, D, E, F, G, H,* and *I* have different spectral pass-bands..

In some implementations, each filter-type *A, B, C, D, E, F, G, H,* and *I* has a spectral pass-band different from the others. In some implementations, the filter-types *A, B, C, D, E, F, G, H,* and *I* are arranged in a 3x3 grid that is repeated across the filter array 201. For example, as illustrated in Figure 4, three filter elements 211a-1, 211b-1, 211c-1 of filter-type *A* are illustrated to show that instances of filter-type *A* are repeated in a uniform distribution across the filter array 201 such that the center-to-center distance *d1* between two filters of the same type is less than 250 microns. In some implementations, the center-to-center distance *d1* between two filters of the same type is less than 100 microns.

Moreover, while nine filter-types are illustrated for example in Figure 4, those skilled in the art will appreciate from the present disclosure that any number of filter types can be used in various implementations. For example, in some implementations 3, 5, 16 or 25 filter-types can be used in various implementations. Additionally and/or alternatively, while a uniform distribution of filter-types has been illustrated and described, those skilled in the art will appreciate from the present disclosure that, in various implementations, one or more filter-types may be distributed across a filter array in a non-uniform distribution. Additionally and/or alternatively, those skilled in the art will also appreciate that "white-light" or transparent filter elements may be included as one of the filter-types in a filter array.

Figure 4 illustrates an advantage of the spectral images of the present disclosure. A single exposure of light 21 from a lens assembly is filtered by filter array 201 to form filtered light 31 that impinges upon sensor 101 and, from this single exposure, multiple images 1337 of the same region 280 of a patient are concurrently made. Figure 4 illustrates a hyperspectral imaging device 132/142/232 comprising a photo-sensor array 101 including a plurality of photo-sensors 111. Each photo-sensor 111 provides a respective output. Hyperspectral imaging device 132/142/232 further comprises a spectral filter array 201 having a plurality of filter elements 211. Each filter element 211 is arranged to filter light 21 received by a respective one or more of the plurality of photo-sensors 111. Each filter element 211 is one of a plurality of filter-types. For instance, in Figure 4, each filter element 211 is one of filter types *A, B, C, D, E, F, G, H,* and *I*, with each respective filter-type characterized by a spectral pass-band different from the other filter-types. An interface module 541 selects one or more subsets of photo-sensor 111 outputs. Each subset of photo-sensor 111 outputs is associated with (receives light exclusively through) a single respective filter-type. For instance, in one such subset are the photo-sensors 111 that are associated with (receive light exclusively from) filter type A, another such subset are the photo-sensors 111 that are associated with filter type *B* and so forth. A control module is configured to generate a hyperspectral data cube 1336 from the one or more sub-sets of photo-sensor outputs by generating a plurality of respective images 1337. In all embodiments, each respective image 1337 in the plurality of images is produced from a single respective sub-set of photo-sensor outputs 111 so that each respective image 1337 in the plurality of images is associated with a particular filter-type. Thus, for example, referring to Figure 4, all the photo-sensors 111 that receive filtered light from filter elements 211 of filter type A are used to form a first image 1337-1, all the photo-sensors 111 that receive filtered light from filter elements 211 of filter type *B* are used to form a second image 1337-2, all the photo-sensors 111 that receive filtered light from filter elements 211 of filter type *C* are used to form a third image 1337-3, and so forth thereby creating a hyperspectral data cube 1336 from the one or more sub-sets of photo-sensor outputs. The hyperspectral data cube 1336 comprises the plurality of images, each image being of the same region of a subject but at a different wavelength or wavelength ranges.

The concept disclosed in Figure 4 is highly advantageous because multiple light exposures do not need to be used to acquire all the images 1337 needed to form the hyperspectral data cube 1336. In some embodiments, a single light exposure is used to concurrently acquire each image 1337. This is made possible because the spatial resolution of the sensor 101 exceeds the resolution necessary for an image 1337. Thus, rather than using all the pixels in the sensor 101 to form each image 1337, the pixels can be divided up in the manner illustrated in Figure 4, for example, using filter plate 201 so that all the images are taken concurrently.

In some implementations, the spectral pass-bands of the filter-elements used in a filter array 201 correspond to a set of narrow spectral ranges used to identify a particular type of spectral signature in an object (*e.g.,* in a tissue of a subject). According to claim 1, an imaging device comprises a filter array 201 containing a first set of filter elements sufficient to distinguish spectral signatures related to a first medical condition from healthy tissue. The filter array 201 of the imaging device further contains a second set of filter elements sufficient to distinguish spectral signatures related to a second medical from healthy tissue. In some implementations, the first set of filter elements and the second set of filter elements may overlap, such that a particular filter element is used for investigation of both types of medical conditions. Accordingly, the imaging device will have a plurality of imaging modalities, each individual imaging modality related to the investigation of a different medical condition.

In all embodiments, each respective image 1337 of the plurality of images is generated by applying an interpolation process to the respective subset of photo-sensor outputs for the one respective filter-type corresponding to the respective image. Such interpolation processes are known in the art.

As with light sources, filter elements 211 can be described in terms of their spectral "bandpass," *e.g.,* the span of component wavelengths allowed to pass through the filter. In some implementations, the bandpass of a filter element 211is defined as the span of component wavelengths at which the filter 211 is at least half as transparent as compared to the characteristic or center wavelength (FWHM). For example, the spectral bandpass of a filter element 211 that is 100% transparent with respect to at least one component wavelength is the span of consecutive component wavelengths at which the filter element is at least 50% transparent. In certain implementations, the bandpass of a filter element 211 can be equivalently expressed in terms of the component wavelengths (*e.g.,* 450-480 nm) or as the width of the bandpass at the central wavelength (*e.g.,* 30 nm at 465 nm or f 15 nm at 465 nm).

A bandpass filter of a filter element 211 can also be described in terms of its "characteristic wavelength," *e.g.,* the wavelength at which the filter is most transparent, or its "center wavelength," *e.g.,* the component wavelength at the midpoint of the spectral bandpass. In certain implementations, the bandpass filter is characterized by both its characteristic or center wavelength and its spectral bandwidth. For example, a bandpass filter with a center wavelength of 340±2 nm, a FWHM bandwidth of 10±2, and a peak transmission (*e.g.,* the maximum percentage transmission within the passband) of 50%, allows at least 25% of each component light having a wavelength from 330±4 nm to 350±4 nm to pass through.

In specific implementations, a filter element 211 is a bandpass filter, *e.g.,* a filter that allows only radiation having a wavelength in a certain range to pass, while blocking passage of other wavelengths. In all embodiments, the FWHM spectral bandpass of a filter element 211 (*e.g*., the size of the passband transmitted through the filter) is no more than 25 nm. In yet other embodiments, the FWHM spectral bandwidth of a filter element 211 is no more than 250 nm, 200 nm, 200 nm, 175 nm, 150 nm, 150 nm, 125 nm, 100 nm, 90 nm, 80 nm, 75 nm, 70 nm, 65 nm, 60 nm, 55 nm, 50 nm, 45 nm, 40 nm, 35 nm, 30 nm, 25 nm, 20 nm, 15 nm, 10 nm, 9 nm, 8 nm, 7 nm, 6 nm, 5 nm, 4 nm, 3 nm, 2 nm, or 1 nm.

In certain implementations, the bandpass filter of a filter element 211 is a narrow pass filter. In all implementations, the narrow pass filter has a FWHM spectral bandwidth of no more than 25 nm, preferably no more than 24 nm, 23 nm, 22 nm, 21 nm, 20 nm, 19 nm, 18 nm, 17 nm, 16 nm, 15 nm, 14 nm, 13 nm, 12 nm, 11 nm, 10 nm, 9 nm, 8 nm, 7 nm, 6nm, 5 nm, 4 nm, 3 nm, 2 nm, or 1 nm.

In some implementations, the filter elements 211, for instance those illustrated in Figure 4, are plurality of bandpass illumination filters having central wavelengths that are separated by at least 10 nm, or at least 15 nm, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, or more.

Figure 5 is a block diagram of an implementation of a single-sensor hyperspectral imaging device 132/142/232 (hereinafter referred to as "imaging device 500" for brevity). While some example features are illustrated in Figure 5, those skilled in the art will appreciate from the present disclosure that various other features have not been illustrated for the sake of brevity and so as not to obscure more pertinent aspects of the example implementations disclosed herein. To that end, the imaging device 500 includes one or more central processing units (CPU) 508, an optional main non-volatile storage unit 540, an optional controller 542, a system memory 514 for storing system control programs, data, and application programs, including programs and data optionally loaded from the non-volatile storage unit 540. In some implementations the non-volatile storage unit 540 includes a memory card, for storing software and data. The storage unit 540 is optionally controlled by the controller 542.

In some implementations, the imaging device 500 optionally includes a user interface 502 including one or more input devices 506 (*e.g.,* a touch screen, buttons, or switches) and/or an optional display 504. Additionally and/or alternatively, in some implementations, the imaging device 500 may be controlled by an external device such as a handheld device, a smartphone (or the like), a tablet computer, a laptop computer, a desktop computer, and/or a server system. To that end, the imaging device 500 includes one or more communication interfaces 512 for connecting to any wired or wireless external device or communication network (*e.g.,* a wide area network such as the Internet) 513. The imaging device 500 includes an internal bus 510 for interconnecting the aforementioned elements. The communication bus 510 may include circuitry (sometimes called a chipset) that interconnects and controls communications between the aforementioned components.

In some implementations, the imaging device 500 communicates with a communication network 513, thereby enabling the imaging device 500 to transmit and/or receive data between mobile communication devices over the communication network, particularly one involving a wireless link, such as cellular, WiFi, ZigBee, BlueTooth, IEEE 802.11b, 802.11a, 802.11g, or 802.11n, *etc.* The communication network can be any suitable communication network configured to support data transmissions. Suitable communication networks include, but are not limited to, cellular networks, wide area networks (WANs), local area networks (LANs), the Internet, IEEE 802.11b, 802.11a, 802.11g, or 802.11n wireless networks, landline, cable line, fiber-optic line, *etc.* The imaging system, depending on an embodiment or desired functionality, can work completely offline by virtue of its own computing power, on a network by sending raw or partially processed data, or both concurrently.

The system memory 514 includes high-speed random access memory, such as DRAM, SRAM, DDR RAM, or other random access solid state memory devices; and typically includes non-volatile memory flash memory devices, or other non-transitory solid state storage devices. The system memory 514 optionally includes one or more storage devices remotely located from the CPU(s) 508. The system memory 514, or alternately the non-transitory memory device(s) within system memory 514, comprises a non-transitory computer readable storage medium.

In some implementations, operation of the imaging device 500 is controlled primarily by an operating system 520, which is executed by the CPU 508. The operating system 320 can be stored in the system memory 314 and/or storage unit 340. In some embodiments, the image device 500 is not controlled by an operating system, but rather by some other suitable combination of hardware, firmware and software.

In some implementations, the system memory 514 includes one or more of a file system 522 for controlling access to the various files and data structures described herein, an illumination software control module 524 for controlling a light source associated and/or integrated with the imaging device 500, a photo-sensor array software control module 528, a sensor data store 531 for storing sensor data 1332 acquired by the photo-sensor array 101/201, a data processing software module 1334 for manipulating the acquired sensor data, a hyperspectral data cube data store 1335 for storing hyperspectral data cube data 1336 assembled from the acquired sensor, and a communication interface software control module 1338 for controlling the communication interface 312 that connects to an external device (*e.g.,* a handheld device, laptop computer, or desktop computer) and/or communication network (*e.g.,* a wide area network such as the Internet).

In some implementations, the acquired sensor data 1332 is arranged and stored by the filter-type associated with each photo-sensor 111 in the photo-sensor array 101. For example, as illustrated in Figure 4, the photo-sensor output data 1332-1 from the photo-sensors associated with filter-type *A* are selectable from the photo-sensor output data, such as photo-sensor output data 1332-K associated with filter-type *I*.

The acquired sensor data 1332 and hyperspectral data cube data 1336 can be stored in a storage module in the system memory 514, and do not need to be concurrently present, depending on which stages of the analysis the imaging device 500 has performed at a given time. In some implementations, prior to imaging a subject and after communicating the acquired sensor data or processed data files thereof, the imaging device 500 contains neither acquired sensor data 1332 nor the hyperspectral data cube data 1336. In some implementations, after imaging a subject and after communicating the acquired sensor data or processed data files thereof, the imaging device 500 retains the acquired sensor data 1332 and/or hyperspectral data cube data 1336 for a period of time (e.g., until storage space is needed, for a predetermined amount of time, etc.).

In some implementations, the programs or software modules identified above correspond to sets of instructions for performing a function described above. The sets of instructions can be executed by one or more processors, *e.g.,* a CPU(s) 508. The above identified software modules or programs (*e.g.,* sets of instructions) need not be implemented as separate software programs, procedures, or modules, and thus various subsets of these programs or modules may be combined or otherwise re-arranged in various embodiments. In some embodiments, the system memory 514 stores a subset of the modules and data structures identified above. Furthermore, the system memory 514 may store additional modules and data structures not described above.

The system memory 514 also includes a spectral library which includes profiles for a plurality of medical conditions, a spectral analyzer software module to compare measured hyperspectral data to a spectral library. The system memory optionally also includes: control modules for additional sensors, information acquired by one or more additional sensors, an image constructor software module for generating a hyperspectral image, a hyperspectral image assembled based on a hyperspectral data cube and optionally fused with information acquired by an additional sensor, a fusion software control module for integrating data acquired by an additional sensor into a hyperspectral data cube, and a display software control module for controlling a built-in display.

While examining a subject and/or viewing hyperspectral images of the subject, a physician can optionally provide input to the image device 500 that modifies one or more parameters upon which a hyperspectral image and/or diagnostic output is based. In some implementations, this input is provided using input device 506. Among other things, the image device can be controlled to modify the spectral portion selected by a spectral analyzer (*e.g.,* to modify a threshold of analytical sensitivity) or to modify the appearance of the image generated by an image assembler (*e.g.,* to switch from an intensity map to a topological rendering).

In some implementations, the imaging device 500 can be instructed to communicate instructions to an imaging subsystem to modify the sensing properties of one of the photo-sensor array 101 and the filter array 201 (*e.g.,* an exposure setting, a frame rate, an integration rate, or a wavelength to be detected). Other parameters can also be modified. For example, the imaging device 500 can be instructed to obtain a wide-view image of the subject for screening purposes, or to obtain a close-in image of a particular region of interest.

In some implementations, the imaging device 500 does not include a controller 542 or storage unit 540. In some such implementations, the memory 514 and CPU 508 are one or more application-specific integrated circuit chips (ASICs) and/or programmable logic devices (e.g. an FGPA - Filed Programmable Gate Array). For example, in some implementations, an ASIC and/or programmed FPGA includes the instructions of the illumination control module 524, photo-sensor array control module 528, the data processing module 534 and/or communication interface control module 538. In some implementations, the ASIC and/or FPGA further includes storage space for the acquired sensor data store 531 and the sensor data 1332 stored therein and/or the hyperspectral data cube data store 1335 and the hyperspectral/multispectral data cubes 1336 stored therein.

In all embodiments, the system memory 514 includes a spectral library and spectral analyzer for comparing hyperspectral data generated by the image device 500 to known spectral patterns associated with various medical conditions. In some implementations, analysis of the acquired hyperspectral data is performed on an external device such as a handheld device, tablet computer, laptop computer, desktop computer, an external server, for example in a cloud computing environment.

In all embodiments, a spectral library includes profiles for a plurality of medical conditions, each of which contain a set of spectral characteristics unique to the medical condition. A spectral analyzer uses the spectral characteristics to determine the probability that a region of the subject corresponding to a measured hyperspectral data cube is afflicted with the medical condition. In some implementations, each profile includes additional information about the condition, *e.g.,* information about whether the condition is malignant or benign, options for treatment, *etc.* In some implementations, each profile includes biological information, *e.g.,* information that is used to modify the detection conditions for subjects of different skin types. In some implementations, the spectral library is stored in a single database. In other implementations, such data is instead stored in a plurality of databases that may or may not all be hosted by the same computer, *e.g.,* on two or more computers addressable by wide area network. In some implementations, the spectral library is electronically stored in the storage unit 540 and recalled using the controller 542 when needed during analysis of hyperspectral data cube data.

In some implementations, the spectral analyzer analyzes a particular spectra derived from hyperspectral data cube data, the spectra having pre-defined spectral ranges (*e.g.,* spectral ranges specific for a particular medical condition), by comparing the spectral characteristics of a pre-determined medical condition to the subject's spectra within the defined spectral ranges. In some implementations, the pre-defined spectral ranges correspond to values of one or more of deoxyhemoglobin levels, oxyhemoglobin levels, total hemoglobin levels, oxygen saturation, oxygen perfusion, hydration levels, total hematocrit levels, melanin levels, and collagen levels of a tissue on a patient (*e.g.,* an area 280a of the body of a subject 280). Performing such a comparison only within defined spectral ranges can both improve the accuracy of the characterization and reduce the computational power needed to perform such a characterization.

According to claim 1, the device is configured to determine the probability that a subject is afflicted with one of at least two medical conditions selected from a group consisting of: venous stasis, cardiac decomposition, respiratory insufficiency, hypovolemia, the progression of diabetes, congestive heart failure, sepsis, dehydration, hypertension, and exposure to chemical or biological agents. In some implementations, a further medical condition is selected from the group consisting of tissue ischemia, ulcer formation, ulcer progression, pressure ulcer formation, pressure ulcer progression, diabetic foot ulcer formation, diabetic foot ulcer progression, venous ulcer disease, infection, shock, hemorrhage, and an inflammatory response.

In some implementations, the spectral analyzer identifies a spectral signature within the hyperspectral data cube that corresponds with a medical condition of the patient. In certain implementations, this is accomplished by identifying a pattern of oxidation or hydration in a tissue associated with a tissue of the patient. In some implementations, the analysis of the hyperspectral data cube includes performing at least one of adjusting the brightness of at least one of the respective digital images in the hyperspectral data cube (e.g., image 1337-1-N at wavelength range No. N), adjusting the contrast of at least one of the respective digital images in the hyperspectral data cube, removing an artifact from at least one of the respective digital images in the hyperspectral data cube, processing one or more sub-pixels of at least one of the respective digital images in the hyperspectral data cube, and transforming a spectral hypercube assembled from a plurality of digital images.

In some implementations, the display 504 which receives an image (e.g., a color image, mono-wavelength image, or hyperspectral/multispectral image) from a display control module, and displays the image. Optionally, the display subsystem also displays a legend that contains additional information. For example, the legend can display information indicating the probability that a region has a particular medical condition, a category of the condition, a probable age of the condition, the boundary of the condition, information about treatment of the condition, information indicating possible new areas of interest for examination, and/or information indicating possible new information that could be useful to obtain a diagnosis, e.g., another test or another spectral area that could be analyzed.

In some implementations, a housing display is built into the housing of the imaging device 500. In an example of such an implementation, a video display in electronic communication with the processor 508 is included. In some implementations, the housing display is a touchscreen display that is used to manipulate the displayed image and/or control the image device 500.

In some implementations, the communication interface 512 comprises a docking station for a mobile device having a mobile device display. A mobile device, such as a smart phone, a personal digital assistant (PDA), an enterprise digital assistant, a tablet computer, an IPOD, a digital camera, or a portable music player, can be connected to the docking station, effectively mounting the mobile device display onto the imaging device 500. Optionally, the mobile device is used to manipulate the displayed image and/or control the image device 500.

In some implementations, the imaging device 500 is configured to be in wired or wireless communication with an external display, for example, on a handheld device, tablet computer, laptop computer, desktop computer, television, IPOD, or projector unit, on which the image is displayed. Optionally, a user interface on the external device is used to manipulate the displayed image and/or control the imaging device 500.

In some implementations, an image can be displayed in real time on the display. The real-time image can be used, for example, to focus an image of the subject, to select an appropriate region of interest, and to zoom the image of the subject in or out. In one embodiment, the real-time image of the subject is a color image captured by an optical detector that is not covered by a detector filter. In some implementations, the imager subsystem comprises an optical detector dedicated to capturing true color images of a subject. In some implementations, the real-time image of the subject is a monowavelength, or narrow-band (*e.g.,* 10-50 nm), image captured by an optical detector covered by a detector filter. In these embodiments, any optical detector covered by a detector filter in the imager subsystem may be used for: (i) resolving digital images of the subject for integration into a hyperspectral data cube; and (ii) resolving narrow-band images for focusing, or otherwise manipulating the optical properties of the imaging device 500.

In some implementations, a hyperspectral image constructed from data collected by the photo-sensor array 101 is displayed on an internal housing display, mounted housing display, or external display. Assembled hyperspectral data (*e.g.,* present in a hyperspectral/multispectral data cube) is used to create a two-dimensional representation of the imaged object or subject, based on one or more parameters. An image constructor module, stored in the imaging system memory or in an external device, constructs an image based on, for example, an analyzed spectra. Specifically, the image constructor creates a representation of information within the spectra. In one example, the image constructor constructs a two-dimensional intensity map in which the spatially-varying intensity of one or more particular wavelengths (or wavelength ranges) within the spectra is represented by a corresponding spatially varying intensity of a visible marker.

In some implementations, the image constructor fuses a hyperspectral image with information obtained from one or more additional sensors. Non-limiting examples of suitable image fusion methods include: band overlay, high-pass filtering method, intensity hue-saturation, principle component analysis, and discrete wavelet transform.

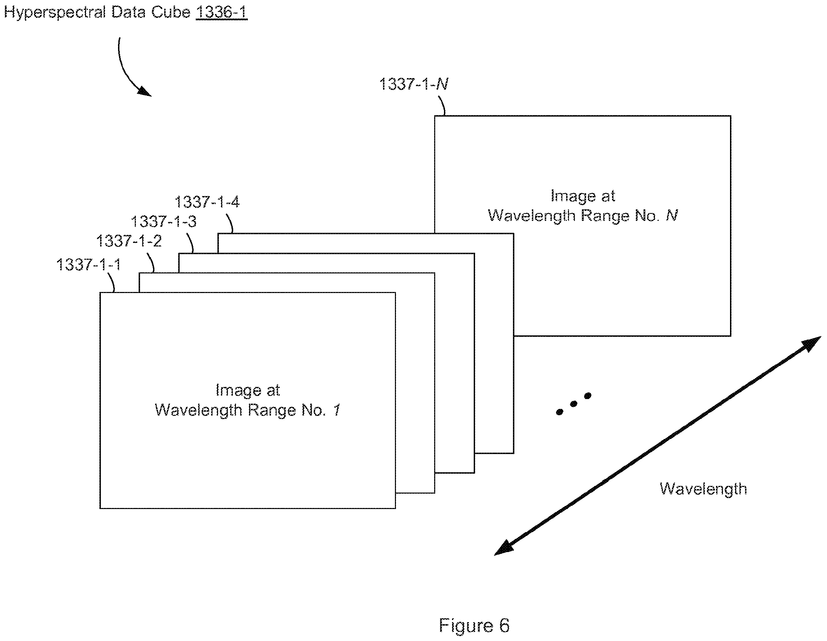

Figure 6 is a schematic illustration of a hyperspectral data cube 1336. Hyperspectral sensors collect information as a set of images, which are referred to herein as hyperspectral data cube planes 1337. Each image 1137 represents a range of the electromagnetic spectrum and is also known as a spectral band. These 'images' 1337 are then combined and form a three-dimensional hyperspectral data cube 1336 for processing and analysis.

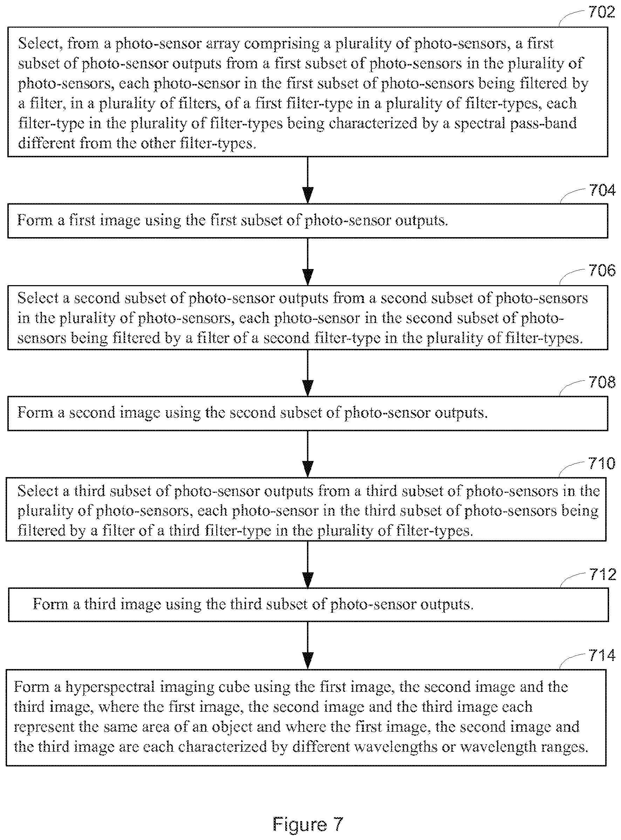

Figure 7 is a flowchart representation of an implementation of a method associated with hyperspectral imaging device, which does not form part of the claimed invention. Specifically, what is illustrated is a method for forming a hyperspectral imaging cube 1336. In the method, at step 702 there is selected, from a photo-sensor array 101 comprising a plurality of photo-sensors 111, a first subset of photo-sensor outputs from a first subset of photo-sensors in the plurality of photo-sensors. Each photo-sensor in the first subset of photo-sensors is filtered by a filter 211, in a plurality of filters, of a first filter-type in a plurality of filter-types. Each filter-type in the plurality of filter-types is characterized by a spectral pass-band different from the other filter-types. For instance, using Figure 4 as a guide, in an example of step 702, each of the photo-sensors 111 that is filtered by a filter 211 of filter type *A* is selected. In this way, the photo-sensor outputs are all from photo-sensors 111 that have received filtered light of the same first wavelength or wavelength range. In step 704 a first image 1337-1 is formed using the first subset of photo-sensor outputs.

In step 706 a second subset of photo-sensor outputs from a second subset of photo-sensors in the plurality of photo-sensors is selected. Each photo-sensor in the second subset of photo-sensors is filtered by a filter of a second filter-type in the plurality of filter-types. For instance, again using Figure 4 as a guide, in an example of step 706, each of the photo-sensors 111 that is filtered by a filter 211 of filter type *B* is selected. In this way, the photo-sensor outputs are all from photo-sensors 111 that have received filtered light of the same second wavelength or wavelength range, this second wavelength or wavelength range being different than the first wavelength or wavelength range. In step 708 a second image 1337-2 is formed using the second subset of photo-sensor outputs.

In step 710 a third subset of photo-sensor outputs from a third subset of photo-sensors in the plurality of photo-sensors is selected. Each photo-sensor in the third subset of photo-sensors is filtered by a filter of a third filter-type in the plurality of filter-types. For instance, again using Figure 4 as a guide, in an example of step 710, each of the photo-sensors 111 that is filtered by a filter 211 of filter type *C* is selected. In this way, the photo-sensor outputs are all from photo-sensors 111 that have received filtered light of the same third wavelength or wavelength range, this third wavelength or wavelength range being different than the first wavelength or wavelength range and also being different from the second wavelength or wavelength range. In step 712 a third image 1337-3 is formed using the third subset of photo-sensor outputs.

In step 714, a hyperspectral imaging cube is formed using the first image 1337-1, the second image 1337-2 and the third image 1337-3 where, as discussed above, the first image, the second image and the third image each represent the same area of an object and where the first image, the second image and the third image are each characterized by different wavelengths or wavelength ranges. This method is highly advantageous. All the images 1337 are taken from the same light exposure. Thus, registration of the images is more accurate and the risk of object movement between images is eliminated. Moreover, there are no moving parts required to affect the filtering and thus the cost to manufacture the spectral imager that performs the disclosed method are much lower than prior art imagers. In some embodiments, all the steps of the method illustrated in Figure 7 are performed by the hyperspectral imager. In some embodiments, the method illustrated in Figure 7 further has additional steps of adding additional images representing still additional wavelengths or wavelength ranges to the hyperspectral imaging cube. In some embodiments, the method illustrated in Figure 7 further has the additional step of displaying all or a portion of the hyperspectral imaging cube. In some embodiments, the display for displaying the hyperspectral image is in a common housing shared by the imager 132/142/232. In all embodiments, steps 704, 708, and 712 are performed on a device that is remote from imager 132/142/232. In all embodiments, steps 704, 708, and 712 are performed by imager 132/142/232.

### Hyperspectral Imaging

Hyperspectral and multispectral imaging are related techniques in larger class of spectroscopy commonly referred to as spectral imaging or spectral analysis. Typically, hyperspectral imaging relates to the acquisition of a plurality of images, each image representing a narrow spectral band collected over a continuous spectral range, for example, 5 or more (*e.g.,* 5, 10, 15, 20, 25, 30, 40, 50, or more) spectral bands having a FWHM bandwidth of 1 nm or more each (e.g., 1 nm, 2 nm, 3 nm, 4 nm, 5 nm, 10 nm, 20 nm or more), covering a contiguous spectral range (e.g., from 400 nm to 800 nm). In contrast, multispectral imaging relates to the acquisition of a plurality of images, each image representing a narrow spectral band collected over a discontinuous spectral range.

For the purposes of the present disclosure, the terms "hyperspectral" and "multispectral" are used interchangeably and refer to a plurality of images, each image representing a narrow spectral band (having a FWHM bandwidth of between 10 nm and 30 nm, between 5 nm and 15 nm, between 5 nm and 50 nm, less than 100 nm, between 1 and 100 nm, *etc*.)*,* whether collected over a continuous or discontinuous spectral range. For example, in some implementations, wavelengths 1-N of a hyperspectral data cube 1336-1 are contiguous wavelengths or spectral bands covering a contiguous spectral range (e.g., from 400 nm to 800 nm). In other implementations, wavelengths 1-N of a hyperspectral data cube 1336-1 are non-contiguous wavelengths or spectral bands covering a non-contiguous spectral ranges (e.g., from 400 nm to 440 nm, from 500 nm to 540 nm, from 600 nm to 680 nm, and from 900 to 950 nm).

As used herein, "narrow spectral range" refers to a continuous span of wavelengths, typically consisting of a FWHM spectral band of no more than about 100 nm. In certain embodiments, narrowband radiation consists of a FWHM spectral band of no more than about 75 nm, 50 nm, 40 nm, 30 nm, 25 nm, 20 nm, 15 nm, 10 nm, 5 nm, 4 nm, 3 nm, 2 nm, 1 nm, or less. In some implementations, wavelengths imaged by the methods and devices disclosed herein are selected from one or more of the visible, near-infrared, short-wavelength infrared, mid-wavelength infrared, long-wavelength infrared, and ultraviolet (UV) spectrums.

By "broadband" it is meant light that includes component wavelengths over a substantial portion of at least one band, *e.g.,* over at least 20%, or at least 30%, or at least 40%, or at least 50%, or at least 60%, or at least 70%, or at least 80%, or at least 90%, or at least 95% of the band, or even the entire band, and optionally includes component wavelengths within one or more other bands. A "white light source" is considered to be broadband, because it extends over a substantial portion of at least the visible band. In certain embodiments, broadband light includes component wavelengths across at least 100 nm of the electromagnetic spectrum. In other embodiments, broadband light includes component wavelengths across at least 150 nm, 200 nm, 250 nm, 300 nm, 400 nm, 500 nm, 600 nm, 700 nm, 800 nm, or more of the electromagnetic spectrum.

By "narrowband" it is meant light that includes components over only a narrow spectral region, e.g., less than 20%, or less than 15%, or less than 10%, or less than 5%, or less than 2%, or less than 1%, or less than 0.5% of a single band. Narrowband light sources need not be confined to a single band, but can include wavelengths in multiple bands. A plurality of narrowband light sources may each individually generate light within only a small portion of a single band, but together may generate light that covers a substantial portion of one or more bands, *e.g.,* may together constitute a broadband light source. In certain embodiments, broadband light includes component wavelengths across no more than 100 nm of the electromagnetic spectrum (*e.g.,* has a spectral bandwidth of no more than 100 nm). In other embodiments, narrowband light has a spectral bandwidth of no more than 90 nm, 80 nm, 75 nm, 70 nm, 60 nm, 50 nm, 40 nm, 30 nm, 25 nm, 20 nm, 15 nm, 10 nm, 5 nm, or less of the electromagnetic spectrum.

As used herein, the "spectral bandwidth" of a light source refers to the span of component wavelengths having an intensity that is at least half of the maximum intensity, otherwise known as "full width at half maximum" (FWHM) spectral bandwidth. Many light emitting diodes (LEDs) emit radiation at more than a single discreet wavelength, and are thus narrowband emitters. Accordingly, a narrowband light source can be described as having a "characteristic wavelength" or "center wavelength," *i.e.,* the wavelength emitted with the greatest intensity, as well as a characteristic spectral bandwidth, *e.g.,* the span of wavelengths emitted with an intensity of at least half that of the characteristic wavelength.

By "coherent light source" it is meant a light source that emits electromagnetic radiation of a single wavelength in phase. Thus, a coherent light source is a type of narrowband light source with a spectral bandwidth of less than 1 nm. Non-limiting examples of coherent light sources include lasers and laser-type LEDs. Similarly, an incoherent light source emits electromagnetic radiation having a spectral bandwidth of more than 1 nm and/or is not in phase. In this regard, incoherent light can be either narrowband or broadband light, depending on the spectral bandwidth of the light.

Examples of suitable broadband light sources 104 include, without limitation, incandescent lights such as a halogen lamp, xenon lamp, a hydrargyrum medium-arc iodide lamp, and a broadband light emitting diode (LED). In some embodiments, a standard or custom filter is used to balance the light intensities at different wavelengths to raise the signal level of certain wavelength or to select for a narrowband of wavelengths. Broadband illumination of a subject is particularly useful when capturing a color image of the subject or when focusing the hyperspectral/multispectral imaging system.

Examples of suitable narrowband, incoherent light sources 104 include, without limitation, a narrow band light emitting diode (LED), a superluminescent diode (SLD) (*see,* Redding B., arVix: 1110.6860 (2011), a random laser, and a broadband light source covered by a narrow band-pass filter. Examples of suitable narrowband, coherent light sources 104 include, without limitation, lasers and laser-type light emitting diodes. While both coherent and incoherent narrowband light sources 104 can be used in the imaging systems described herein, coherent illumination is less well suited for full-field imaging due to speckle artifacts that corrupt image formation (*see,* Oliver, B.M., Proc IEEE 51, 220-221 (1963)).

### Hyperspectral Medical Imaging

The disclosure provides systems and methods useful for hyperspectral/multispectral medical imaging (HSMI). HSMI relies upon distinguishing the interactions that occur between light at different wavelengths and components of the human body, especially components located in or just under the skin. For example, it is well known that deoxyhemoglobin absorbs a greater amount of light at 700 nm than does water, while water absorbs a much greater amount of light at 1200 nm, as compared to deoxyhemoglobin. By measuring the absorbance of a two-component system consisting of deoxyhemoglobin and water at 700 nm and 1200 nm, the individual contribution of deoxyhemoglobin and water to the absorption of the system, and thus the concentrations of both components, can readily be determined. By extension, the individual components of more complex systems (e.g., human skin) can be determined by measuring the absorption of a plurality of wavelengths of light reflected or backscattered off of the system.

The particular interactions between the various wavelengths of light measured by hyperspectral/multispectral imaging and each individual component of the system (e.g., skin) produces hyperspectral/multispectral signature, when the data is constructed into a hyperspectral/multispectral data cube. Specifically, different regions (e.g., different ROI on a single subject or different ROI from different subjects) interact differently with light depending on the presence of, e.g., a medical condition in the region, the physiological structure of the region, and/or the presence of a chemical in the region. For example, fat, skin, blood, and flesh all interact with various wavelengths of light differently from one another. A given type of cancerous lesion interacts with various wavelengths of light differently from normal skin, from non-cancerous lesions, and from other types of cancerous lesions. Likewise, a given chemical that is present (e.g., in the blood, or on the skin) interacts with various wavelengths of light differently from other types of chemicals. Thus, the light obtained from each illuminated region of a subject has a spectral signature based on the characteristics of the region, which signature contains medical information about that region.

The structure of skin, while complex, can be approximated as two separate and structurally different layers, namely the epidermis and dermis. These two layers have very different scattering and absorption properties due to differences of composition. The epidermis is the outer layer of skin. It has specialized cells called melanocytes that produce melanin pigments. Light is primarily absorbed in the epidermis, while scattering in the epidermis is considered negligible. For further details, see G.H. Findlay, "Blue Skin," British Journal of Dermatology 83(1), 127-134 (1970).

The dermis has a dense collection of collagen fibers and blood vessels, and its optical properties are very different from that of the epidermis. Absorption of light of a bloodless dermis is negligible. However, blood-born pigments like oxy- and deoxyhemoglobin and water are major absorbers of light in the dermis. Scattering by the collagen fibers and absorption due to chromophores in the dermis determine the depth of penetration of light through skin.

Light used to illuminate the surface of a subject will penetrate into the skin. The extent to which the light penetrates will depend upon the wavelength of the particular radiation. For example, with respect to visible light, the longer the wavelength, the farther the light will penetrate into the skin. For example, only about 32% of 400 nm violet light penetrates into the dermis of human skin, while greater than 85% of 700 nm red light penetrates into the dermis or beyond (see, Capinera J.L., Encyclopedia of Entomology, 2nd Edition, Springer Science (2008) at page 2854). For purposes of the present disclosure, when referring to "illuminating a tissue," "reflecting light off of the surface," and the like, it is meant that radiation of a suitable wavelength for detection is backscattered from a tissue of a subject, regardless of the distance into the subject the light travels. For example, certain wavelengths of infra-red radiation penetrate below the surface of the skin, thus illuminating the tissue below the surface of the subject.

Briefly, light from the illuminator(s) on the systems described herein penetrates the subject's superficial tissue and photons scatter in the tissue, bouncing inside the tissue many times. Some photons are absorbed by oxygenated hemoglobin molecules at a known profile across the spectrum of light. Likewise for photons absorbed by de-oxygenated hemoglobin molecules. The images resolved by the optical detectors consist of the photons of light that scatter back through the skin to the lens subsystem. In this fashion, the images represent the light that is not absorbed by the various chromophores in the tissue or lost to scattering within the tissue. In some embodiments, light from the illuminators that does not penetrate the surface of the tissue is eliminated by use of polarizers. Likewise, some photons bounce off the surface of the skin into air, like sunlight reflecting off a lake.

Accordingly, different wavelengths of light may be used to examine different depths of a subject's skin tissue. Generally, high frequency, short-wavelength visible light is useful for investigating elements present in the epidermis, while lower frequency, long-wavelength visible light is useful for investigating both the epidermis and dermis. Furthermore, certain infra-red wavelengths are useful for investigating the epidermis, dermis, and subcutaneous tissues.

In the visible and near-infrared (VNIR) spectral range and at low intensity irradiance, and when thermal effects are negligible, major light-tissue interactions include reflection, refraction, scattering and absorption. For normal collimated incident radiation, the regular reflection of the skin at the air-tissue interface is typically only around 4%-7% in the 250-3000 nanometer (nm) wavelength range. For further details, see R.R. Anderson and J.A. Parrish, "The optics of human skin," Journal of Investigative Dermatology 77(1), 13-19 (1981), the content of which is hereby incorporated by reference in its entirety for all purposes. When neglecting the air-tissue interface reflection and assuming total diffusion of incident light after the stratum corneum layer, the steady state VNIR skin reflectance can be modeled as the light that first survives the absorption of the epidermis, then reflects back toward the epidermis layer due the isotropic scattering in the dermis layer, and then finally emerges out of the skin after going through the epidermis layer again.

Accordingly, the systems and methods described herein can be used to diagnose and characterize a wide variety of medical conditions. In all embodiments, oxygen saturation is determined and, optionally, the concentration of one or more additional skin or blood components, is determined in order to evaluate a medical condition in a patient. Non-limiting examples of further components useful for medical evaluation include: deoxyhemoglobin levels, oxyhemoglobin levels, total hemoglobin levels, oxygen perfusion, hydration levels, total hematocrit levels, melanin levels, collagen levels, and bilirubin levels. Likewise, the pattern, gradient, or change over time of a skin or blood component can be used to provide information on the medical condition of the patient.

Non-limiting examples of conditions that can be evaluated by hyperspectral/multispectral imaging, include: tissue ischemia, ulcer formation, ulcer progression, pressure ulcer formation, pressure ulcer progression, diabetic foot ulcer formation, diabetic foot ulcer progression, venous stasis, venous ulcer disease, infection, shock, cardiac decompensation, respiratory insufficiency, hypovolemia, the progression of diabetes, congestive heart failure, sepsis, dehydration, hemorrhage, hypertension, exposure to a chemical or biological agent, and an inflammatory response.

The systems and methods described herein are used to evaluate tissue oximetery by determining oxygen saturation. Correspondingly, medical conditions relating to patient health derived from oxygen measurements in the superficial vasculature can be evaluated. In certain embodiments, the systems and methods described herein further allow for the measurement of oxygenated hemoglobin, deoxygenated hemoglobin, and oxygen perfusion. Processing of these data provide information to assist a physician with, for example, diagnosis, prognosis, assignment of treatment, assignment of surgery, and the execution of surgery for conditions such as critical limb ischemia, diabetic foot ulcers, pressure ulcers, peripheral vascular disease, surgical tissue health, etc.

In one embodiment, the systems and methods described herein are used to evaluate diabetic and pressure ulcers. Development of a diabetic foot ulcer is commonly a result of a break in the barrier between the dermis of the skin and the subcutaneous fat that cushions the foot during ambulation. This rupture can lead to increased pressure on the dermis, resulting in tissue ischemia and eventual death, and ultimately manifesting in the form of an ulcer (Frykberg R.G. et al., Diabetes Care 1998;21(10):1714-9). Measurement of oxyhemoglobin, deoxyhemoglobin, and/or oxygen saturation levels by hyperspectral/multispectral imaging can provide medical information regarding, for example: a likelihood of ulcer formation at an ROI, diagnosis of an ulcer, identification of boundaries for an ulcer, progression or regression of ulcer formation, a prognosis for healing of an ulcer, the likelihood of amputation resulting from an ulcer. Further information on hyperspectral/multispectral methods for the detection and characterization of ulcers, e.g., diabetic foot ulcers, are found in U.S. Patent Application Publication No. 2007/0038042, and Nouvong A. et al., Diabetes Care. 2009 Nov; 32(11):2056-61.

Other examples of medical conditions include, but are not limited to: tissue viability (e.g., whether tissue is dead or living, and/or whether it is predicted to remain living); tissue ischemia; malignant cells or tissues (e.g., delineating malignant from benign tumors, dysplasias, precancerous tissue, metastasis); tissue infection and/or inflammation; and/or the presence of pathogens (e.g., bacterial or viral counts). Some embodiments include differentiating different types of tissue from each other, for example, differentiating bone from flesh, skin, and/or vasculature. Some embodiments exclude the characterization of vasculature.

In yet other embodiments, the systems and methods provided herein can be used during surgery, for example to determine surgical margins, evaluate the appropriateness of surgical margins before or after a resection, evaluate or monitor tissue viability in near-real time or real-time, or to assist in image-guided surgery. For more information on the use of hyperspectral/multispectral imaging during surgery, see, Holzer M.S. et al., J Urol. 2011 Aug; 186(2):400-4; Gibbs-Strauss S.L. et al., Mol Imaging. 2011 Apr; 10(2):91-101; and Panasyuk S.V. et al., Cancer Biol Ther. 2007 Mar; 6(3) 439-46.

For more information on the use of hyperspectral/multispectral imaging in medical assessments, see, for example: Chin J.A. et al., J Vase Surg. 2011 Dec; 54(6):1679-88; Khaodhiar L. et al., Diabetes Care 2007;30:903-910; Zuzak K.J. et al., Anal Chem. 2002 May 1;74(9):2021-8; Uhr J.W. et al., Transl Res. 2012 May; 159(5):366-75; Chin M.S. et al., J Biomed Opt. 2012 Feb; 17(2):026010; Liu Z. et al., Sensors (Basel). 2012; 12(1):162-74; Zuzak K.J. et al., Anal Chem. 2011 Oct 1;83(19):7424-30; Palmer G.M. et al., J Biomed Opt. 2010 Nov-Dec; 15(6):066021; Jafari-Saraf and Gordon, Ann Vase Surg. 2010 Aug; 24(6):741-6; Akbari H. et al., IEEE Trans Biomed Eng. 2010 Aug; 57(8):2011-7; Akbari H. et al., Conf Proc IEEE Eng Med Biol Soc. 2009:1461-4; Akbari H. et al., Conf Proc IEEE Eng Med Biol Soc. 2008:1238-41; Chang S.K. et al., Clin Cancer Res. 2008 Jul 1;14(13):4146-53; Siddiqi A.M. et al., Cancer. 2008 Feb 25;114(1):13-21; Liu Z. et al., Appl Opt. 2007 Dec 1;46(34):8328-34; Zhi L. et al., Comput Med Imaging Graph. 2007 Dec; 31(8):672-8; Khaodhiar L. et al., Diabetes Care. 2007 Apr; 30(4):903-10; Ferris D.G. et al., J Low Genit Tract Dis. 2001 Apr; 5(2):65-72; Greenman R.L. et al., Lancet. 2005 Nov 12;366(9498):1711-7; Sorg B.S. et al., J Biomed Opt. 2005 Jul-Aug; 10(4):44004; Gillies R. et al., and Diabetes Technol Ther. 2003;5(5): 847-55.

In yet other embodiments, the systems and methods provided herein can be used during surgery, for example to determine surgical margins, evaluate the appropriateness of surgical margins before or after a resection, evaluate or monitor tissue viability in near-real time or real-time, or to assist in image-guided surgery. For more information on the use of hyperspectral/multispectral imaging during surgery, *see,* Holzer M.S. et al., J Urol. 2011 Aug; 186(2):400-4; Gibbs-Strauss S.L. et al., Mol Imaging. 2011 Apr; 10(2):91-101; and Panasyuk S.V. et al., Cancer Biol Ther. 2007 Mar; 6(3) 439-46.

In some implementations, the systems and methods provided herein are useful for other hyperspectral/multispectral applications such as satellite imaging (*e.g.,* for geological sensing of minerals, and agricultural imaging), remote chemical imaging, and environmental monitoring. For example, a spectral filter array 201 having a plurality of filter elements 211 and a photo-sensor array 101 including a plurality of photo-sensors 111 can be mounted inside a satellite or other telescopic apparatus for remote hyperspectral/multispectral imaging.

The terminology used herein is for the purpose of describing particular embodiments only and is not intended to be limiting of the claims. As used in the description of the embodiments and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

It will also be understood that, although the terms "first," "second," etc. may be used herein to describe various elements, these elements should not be limited by these terms. These terms are only used to distinguish one element from another. For example, a first contact could be termed a second contact, and, similarly, a second contact could be termed a first contact, which changing the meaning of the description, so long as all occurrences of the "first contact" are renamed consistently and all occurrences of the second contact are renamed consistently. The first contact and the second contact are both contacts, but they are not the same contact.

As used herein, the term "if" may be construed to mean "when" or "upon" or "in response to determining" or "in accordance with a determination" or "in response to detecting," that a stated condition precedent is true, depending on the context. Similarly, the phrase "if it is determined [that a stated condition precedent is true]" or "if [a stated condition precedent is true]" or "when [a stated condition precedent is true]" may be construed to mean "upon determining" or "in response to determining" or "in accordance with a determination" or "upon detecting" or "in response to detecting" that the stated condition precedent is true, depending on the context.

The foregoing description, for purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the invention and its practical applications, to thereby enable others skilled in the art to best utilize the invention and various embodiments with various modifications as are suited to the particular use contemplated.

## Claims

1. A hyperspectral imaging device comprising:
a photo-sensor array including a plurality of photo-sensors, each photo-sensor in the plurality of photo-sensors providing a respective output;
a spectral filter array having a plurality of filter elements, wherein:
the spectral filter array includes a first set of filter elements for distinguishing spectral signatures related to a first medical condition and a second set of filter elements for distinguishing spectral signatures related to a second medical condition, wherein the first and second medical conditions are selected from a group consisting of venous stasis, cardiac decomposition, respiratory insufficiency, hypovolemia, the progression of diabetes, congestive heart failure, sepsis, dehydration, hypertension, and exposure to chemical or biological agents,
each filter element is arranged to filter light received by a respective one or more of the plurality of photo-sensors,
each filter element is one of a plurality of filter-types,
each filter-type is **characterized by** a spectral pass-band different from the other filter-types,
each filter element is a narrow pass filter having a full-width at half-maximum spectral bandwidth of no more than 25 nm, and
the plurality of filter elements comprises a first filter element and a second filter element of the same filter-type, wherein a center-to-center distance between the first filter element and the second filter element is less than 250 microns;
an interface module to select a plurality of subsets of photo-sensor outputs, wherein each subset of photo-sensor outputs is associated with a single respective filter-type arranged in the spectral filter;
a control module configured to:
capture single frame image data of a tissue of a subject by controlling the exposure of the combination of the photo-sensor array and spectral filter array to light, and
generate a hyperspectral data cube from the plurality of subsets of photo-sensor outputs by generating a plurality of images, wherein:
each respective image in the plurality of images is produced by applying an interpolation process to a single corresponding subset of photo-sensor outputs in the plurality of photo-sensor outputs so that each respective image is associated with a corresponding filter-type in the plurality of filter-types arranged in the spectral filter, and
the hyperspectral data cube is generated from the single frame image data of the tissue of the subject;
a data processing module configured to determine, using the hyperspectral data cube generated from the single frame image data of the tissue of the subject, concentration of one or more skin or blood components to evaluate tissue oximetry, wherein the one or more skin or blood components comprise oxygen saturation; and
a system memory including a spectral library and a spectral analyzer for comparing the hyperspectral data cube to spectral patterns associated with a plurality of medical conditions, wherein:
the spectral library includes profiles for the plurality of medical conditions, each of which contain a set of spectral characteristics unique to the medical condition, and
the spectral analyzer uses the spectral characteristics to determine the probability that a region of the subject corresponding to a measured hyperspectral data cube is afflicted with one of the first and second medical conditions.

2. The hyperspectral imaging device according to claim 1, wherein the plurality of filter elements comprises a first filter element and a second filter element of the same filter-type and wherein a center-to-center distance between the first filter element and the second filter element is less than 150 microns.

3. The hyperspectral imaging device according to any one of claims 1-2, wherein the filter elements of a first filter-type in the plurality of filter-types are spatially distributed across the spectral filter array in a uniform distribution throughout the spectral filter array.

4. The hyperspectral imaging device according to any one of claims 1-3, wherein a spatial distribution of the filter elements in the plurality of filter elements is **characterized by** a repeating pattern of one or more filter-types.

5. The hyperspectral imaging device according to any one of claims 1-4, wherein the plurality of filter-types includes at least three filter-types.

6. The hyperspectral imaging device according to any one of claims 1-5, wherein the interface module comprises circuitry configured to select the one or more subsets of photo-sensor outputs.

7. The hyperspectral imaging device according to any one of claims 1-6, wherein the interface module comprises:
a plurality of registers configured to receive the output of the photo-sensor array; and
wherein the control module is further configured to:
identify which registers in the plurality of registers correspond to filter elements of a particular filter-type in the plurality of filter-types using a look-up table; and
select one or more subsets of photo-sensor outputs from the plurality of registers based on the identification of the registers that correspond to filter elements of the particular filter-type.

8. The hyperspectral imaging device of claim 7, wherein the control module is also operable to bundle photo-sensor outputs for the particular filter-type into data packets, wherein the data packets include at least the register values of the registers that include data for the particular filter-type.

9. The hyperspectral imaging device according to any one of claims 1-8, wherein the concentration of one or more skin or blood components is determined to evaluate a diabetic ulcer.

10. The hyperspectral imaging device according to any one of claims 1-9, wherein the concentration of one or more skin or blood components is determined to evaluate a pressure ulcer.

11. The hyperspectral imaging device according to any one of claims 1-10, wherein each filter element in the plurality of filter elements is arranged to filter light received by a respective one photo-sensor in the plurality of photo-sensors, wherein each photo-sensor in the plurality of photo-sensors is a single pixel.

12. The hyperspectral imaging device according to any one of claims 1-11, wherein one or more filter-types in the plurality of filter-types are distributed across the spectral filter array in a non-uniform distribution.

## Patentansprüche

1. Hyperspektrale Abbildungsvorrichtung, die Folgendes umfasst:
ein Fotosensor-Array, das eine Vielzahl von Fotosensoren beinhaltet, wobei jeder Fotosensor in der Vielzahl von Fotosensoren einen entsprechende Ausgabe bereitstellt;
ein Spektralfilter-Array, das eine Vielzahl von Filterelementen aufweist, wobei:
das Spektralfilter-Array einen ersten Satz von Filterelementen zur Unterscheidung von Spektralsignaturen, die mit einem ersten medizinischen Zustand in Beziehung stehen, und einen zweiten Satz von Filterelementen zur Unterscheidung von Spektralsignaturen, die mit einem zweiten medizinischen Zustand in Beziehung stehen, umfasst, wobei der erste und der zweite medizinische Zustand aus einer Gruppe ausgewählt sind, die aus venöser Stase, kardialer Zersetzung, respiratorischer Insuffizienz, Hypovolämie, dem Fortschreiten von Diabetes, kongestiver Herzinsuffizienz, Sepsis, Dehydrierung, Bluthochdruck und der Exposition gegenüber chemischen oder biologischen Wirkstoffen besteht,
jedes Filterelement angeordnet ist, um Licht zu filtern, das von einem oder mehreren der Vielzahl von Fotosensoren empfangen wird,
jedes Filterelement ein Filtertyp aus einer Vielzahl von Filtertypen ist,
jeder Filtertyp durch einen spektralen Durchlassbereich gekennzeichnet ist, der verschieden ist von dem anderer Filtertypen,
jedes Filterelement ein schmaler Durchlassfilter mit einer spektralen FWHM-Bandbreite (FWHM: Full Width at Half Maximum) von höchstens 25 nm ist, und
die Vielzahl von Filterelementen ein erstes Filterelement und ein zweites Filterelement desselben Filtertyps umfasst, wobei der Abstand von Mitte zu Mitte zwischen dem ersten Filterelement und dem zweiten Filterelement weniger als 250 Mikrometer beträgt;
ein Schnittstellenmodul zum Auswählen einer Vielzahl von Teilmengen von Fotosensorausgängen, wobei jede Teilmenge von Fotosensorausgängen mit einem einzelnen jeweiligen Filtertyp verbunden ist, der in dem Spektralfilter angeordnet ist;
ein Steuermodul, das konfiguriert ist zum:
Erfassen von Einzelbilddaten von Gewebe einer Versuchsperson durch Steuern der Belichtung der Kombination aus der Fotosensoranordnung und der Spektralfilteranordnung, und
Erzeugen eines hyperspektralen Datenwürfels aus der Vielzahl von Teilmengen von Fotosensorausgaben durch Erzeugen einer Vielzahl von Bildern, wobei:
jedes jeweilige Bild in der Vielzahl von Bildern durch Anwenden eines Interpolationsprozesses auf eine einzelne entsprechende Teilmenge von Fotosensorausgängen in der Vielzahl von Fotosensorausgängen erzeugt wird, so dass jedes jeweilige Bild mit einem entsprechenden Filtertyp in der Vielzahl von Filtertypen, die in dem Spektralfilter angeordnet sind, assoziiert ist, und
der hyperspektrale Datenwürfel aus den Einzelbilddaten des Gewebes der Versuchsperson erzeugt wird;
ein Datenverarbeitungsmodul, das konfiguriert ist, um unter Verwendung des hyperspektralen Datenwürfels, der aus den Einzelbild-Bilddaten des Gewebes der Versuchsperson erzeugt wurde, die Konzentration einer oder mehrerer Haut- oder Blutkomponenten zu bestimmen, um die Gewebeoximetrie zu bewerten, wobei die eine oder mehreren Haut- oder Blutkomponenten die Sauerstoffsättigung umfassen; und
einen Systemspeicher mit einer Spektralbibliothek und einem Spektralanalysator zum Vergleichen des hyperspektralen Datenwürfels mit spektralen Mustern, die mit einer Vielzahl von medizinischen Zuständen assoziiert sind, wobei:
die spektrale Bibliothek Profile für die Vielzahl von medizinischen Zuständen beinhaltet, von denen jeder einen Satz von spektralen Merkmalen enthält, die für den medizinischen Zustand einzigartig sind, und
der Spektralanalysator die spektralen Eigenschaften verwendet, um die Wahrscheinlichkeit zu bestimmen, dass eine Region des Subjekts, die einem gemessenen hyperspektralen Datenwürfel entspricht, von dem ersten und zweiten medizinischen Zustand betroffen ist.

2. Hyperspektrale Abbildungsvorrichtung nach Anspruch 1, wobei die Vielzahl von Filterelementen ein erstes Filterelement und ein zweites Filterelement desselben Filtertyps umfasst und wobei der Abstand von Mitte zu Mitte zwischen dem ersten Filterelement und dem zweiten Filterelement weniger als 150 Mikrometer beträgt.

3. Hyperspektrale Abbildungsvorrichtung nach einem der Ansprüche 1-2, wobei die Filterelemente eines ersten Filtertyps in der Vielzahl der Filtertypen räumlich über das Spektralfilter-Array in einer gleichmäßigen Verteilung über das Spektralfilter-Array verteilt sind.

4. Hyperspektrale Abbildungsvorrichtung nach einem der Ansprüche 1-3, wobei die räumliche Verteilung der Filterelemente in der Vielzahl von Filterelementen durch ein sich wiederholendes Muster von einem oder mehreren Filtertypen gekennzeichnet ist.

5. Hyperspektrale Abbildungsvorrichtung nach einem der Ansprüche 1-4, wobei die Vielzahl der Filtertypen mindestens drei Filtertypen umfasst.

6. Hyperspektrale Abbildungsvorrichtung nach einem der Ansprüche 1-5, wobei das Schnittstellenmodul eine Schaltung umfasst, die konfiguriert ist, um die eine oder mehreren Untergruppen von Fotosensorausgaben auszuwählen.

7. Hyperspektrale Abbildungsvorrichtung nach einem der Ansprüche 1-6, wobei das Schnittstellenmodul Folgendes umfasst:
eine Vielzahl von Registern, die konfiguriert sind, um die Ausgaben des Fotosensor-Arrays zu empfangen; und
wobei das Steuermodul ferner konfiguriert ist zum:
Identifizieren, welche Register in der Vielzahl von Registern Filterelementen eines bestimmten Filtertyps in der Vielzahl von Filtertypen entsprechen, unter Verwendung einer Nachschlagetabelle; und
Auswahl einer oder mehrerer Teilmengen von Fotosensorausgaben aus der Vielzahl von Registern basierend auf der Identifizierung der Register, die Filterelementen des bestimmten Filtertyps entsprechen.

8. Hyperspektrale Abbildungsvorrichtung nach Anspruch 7, wobei das Steuermodul auch betreibbar ist, um Fotosensorausgaben für den bestimmten Filtertyp in Datenpakete zu bündeln, wobei die Datenpakete wenigstens die Registerwerte der Register enthalten, die Daten für den bestimmten Filtertyp enthalten.

9. Hyperspektrale Abbildungsvorrichtung nach einem der Ansprüche 1-8, wobei die Konzentration eines oder mehrerer Haut- oder Blutbestandteile bestimmt wird, um ein diabetisches Geschwür zu bewerten.

10. Hyperspektrale Abbildungsvorrichtung nach einem der Ansprüche 1-9, wobei die Konzentration eines oder mehrerer Haut- oder Blutbestandteile bestimmt wird, um ein Druckgeschwür zu bewerten.

11. Hyperspektrale Abbildungsvorrichtung nach einem der Ansprüche 1-10, wobei jedes Filterelement in der Vielzahl von Filterelementen so angeordnet ist, dass es Licht filtert, das von einem entsprechenden Fotosensor in der Vielzahl von Fotosensoren empfangen wird, wobei jeder Fotosensor in der Vielzahl von Fotosensoren ein einzelnes Pixel ist.

12. Hyperspektrale Abbildungsvorrichtung nach einem der Ansprüche 1-11, wobei ein oder mehrere Filtertypen aus der Vielzahl der Filtertypen in einer ungleichmäßigen Verteilung über die Spektralfilteranordnung verteilt sind.

## Revendications

1. Dispositif d'imagerie hyperspectrale comprenant :
un réseau de photodétecteurs incluant une pluralité de photodétecteurs, chaque photodétecteur de la pluralité de photodétecteurs fournissant une sortie respective ;
un réseau de filtres spectraux ayant une pluralité d'éléments filtrants, dans lequel :
le réseau de filtres spectraux inclut un premier ensemble d'éléments filtrants permettant de distinguer les signatures spectrales liées à un premier état pathologique et un deuxième ensemble d'éléments filtrants permettant de distinguer les signatures spectrales liées à un deuxième état pathologique, dans lequel le premier et le deuxième état pathologique sont choisis dans un groupe comprenant la stase veineuse, la décomposition cardiaque, l'insuffisance respiratoire, l'hypovolémie, la progression du diabète, l'insuffisance cardiaque congestive, la septicémie, la déshydratation, l'hypertension et l'exposition à des agents chimiques ou biologiques,
chaque élément filtrant est conçu pour filtrer la lumière reçue par un ou plusieurs respectif de la pluralité de photodétecteurs,
chaque élément filtrant est l'un d'une pluralité de types de filtres,
chaque type de filtre est **caractérisé par** une bande passante spectrale différente des autres types de filtres,
chaque élément filtrant est un filtre à passage étroit dont la largeur de bande spectrale à mi-maximum ne dépasse pas 25 nm, et
la pluralité d'éléments filtrants comprend un premier élément filtrant et un deuxième élément filtrant du même type de filtre, dans lequel la distance centre à centre entre le premier élément filtrant et le deuxième élément filtrant est inférieure à 250 microns ;
un module d'interface pour sélectionner une pluralité de sous-ensembles de sorties de photodétecteurs, dans lequel chaque sous-ensemble de sorties de photodétecteurs est associé à un seul type de filtre respectif disposé dans le filtre spectral ;
un module de commande configuré pour :
capturer des données d'image de trame unique d'un tissu d'un sujet en contrôlant l'exposition à la lumière de la combinaison du réseau de photodétecteurs et du réseau de filtres spectraux, et
générer un cube de données hyperspectrales à partir de la pluralité de sous-ensembles de sorties de photodétecteurs en générant une pluralité d'images, dans lequel :
chaque image respective de la pluralité d'images est produite en appliquant un processus d'interpolation à un sous-ensemble correspondant unique de sorties de photodétecteurs dans la pluralité de sorties de photodétecteurs, de sorte que chaque image respective est associée à un type de filtre correspondant dans la pluralité de types de filtres disposés dans le filtre spectral, et
le cube de données hyperspectrales est généré à partir des données d'image de trame unique du tissu du sujet ;
un module de traitement des données configuré pour déterminer, à l'aide du cube de données hyperspectrales généré à partir des données d'image de trame unique du tissu du sujet, la concentration d'un ou plusieurs composants de la peau ou du sang pour évaluer l'oxymétrie du tissu, dans lequel les un ou plusieurs composants de la peau ou du sang comprennent la saturation en oxygène ; et
une mémoire système incluant une bibliothèque spectrale et un analyseur spectral pour comparer le cube de données hyperspectrales à des modèles spectraux associés à une pluralité d'états pathologiques, dans lequel :
la bibliothèque spectrale inclut des profils pour la pluralité d'états pathologiques, chacun d'eux contenant un ensemble de caractéristiques spectrales propres à l'état pathologique, et
l'analyseur spectral utilise les caractéristiques spectrales pour déterminer la probabilité qu'une région du sujet correspondant à un cube de données hyperspectrales mesuré soit atteinte de l'une du premier et du deuxième état pathologique.

2. Dispositif d'imagerie hyperspectrale selon la revendication 1, dans lequel la pluralité d'éléments filtrants comprend un premier élément filtrant et un deuxième élément filtrant du même type de filtre et dans lequel la distance centre à centre entre le premier élément filtrant et le deuxième élément filtrant est inférieure à 150 microns.

3. Dispositif d'imagerie hyperspectrale selon l'une quelconque des revendications 1 à 2, dans lequel les éléments filtrants d'un premier type de filtre dans la pluralité de types de filtres sont répartis dans l'espace à travers le réseau de filtres spectraux dans une distribution uniforme à travers le réseau de filtres spectraux.

4. Dispositif d'imagerie hyperspectrale selon l'une quelconque des revendications 1 à 3, dans lequel la distribution spatiale des éléments filtrants dans la pluralité d'éléments filtrants est **caractérisée par** un motif répétitif d'un ou plusieurs types de filtres.

5. Dispositif d'imagerie hyperspectrale selon l'une quelconque des revendications 1 à 4, dans lequel la pluralité de types de filtres inclut au moins trois types de filtres.

6. Dispositif d'imagerie hyperspectrale selon l'une quelconque des revendications 1 à 5, dans lequel le module d'interface comprend des circuits configurés pour sélectionner les un ou plusieurs sous-ensembles de sorties de photodétecteurs.

7. Dispositif d'imagerie hyperspectrale selon l'une quelconque des revendications 1 à 6, dans lequel le module d'interface comprend :
une pluralité de registres configurés pour recevoir la sortie du réseau de photodétecteurs ; et dans lequel le module de commande est en outre configuré pour :
identifier les registres de la pluralité de registres correspondant aux éléments filtrants d'un type de filtre particulier dans la pluralité de types de filtres à l'aide d'une table de recherche ; et
sélectionner un ou plusieurs sous-ensembles de sorties de photodétecteurs dans la pluralité de registres sur la base de l'identification des registres qui correspondent aux éléments filtrants du type de filtre particulier.

8. Dispositif d'imagerie hyperspectrale selon la revendication 7, dans lequel le module de commande est également capable de regrouper les sorties des photodétecteurs pour le type de filtre particulier en paquets de données, dans lequel les paquets de données incluent au moins les valeurs des registres qui incluent des données pour le type de filtre particulier.

9. Dispositif d'imagerie hyperspectrale selon l'une quelconque des revendications 1 à 8, dans lequel la concentration d'un ou plusieurs composants de la peau ou du sang est déterminée pour évaluer un ulcère diabétique.

10. Dispositif d'imagerie hyperspectrale selon l'une quelconque des revendications 1 à 9, dans lequel la concentration d'un ou plusieurs composants de la peau ou du sang est déterminée pour évaluer une escarre.

11. Dispositif d'imagerie hyperspectrale selon l'une quelconque des revendications 1 à 10, dans lequel chaque élément filtrant de la pluralité d'éléments filtrants est conçu pour filtrer la lumière reçue par un photodétecteur respectif de la pluralité de photodétecteurs, dans lequel chaque photodétecteur de la pluralité de photodétecteurs est un pixel unique.

12. Dispositif d'imagerie hyperspectrale selon l'une quelconque des revendications 1 à 11, dans lequel un ou plusieurs types de filtres de la pluralité de types de filtres sont répartis sur le réseau de filtres spectraux selon une distribution non uniforme.
